(19) Europäisches Patentamt
European Patent Office
Office européen des brevets

(11) EP 1 298 995 B1

(12) EUROPÄISCHE PATENTSCHRIFT

(45) Veröffentlichungstag und Bekanntmachung des Hinweises auf die Patenterteilung:
**08.02.2006 Patentblatt 2006/06**

(21) Anmeldenummer: **01943517.1**

(22) Anmeldetag: **18.06.2001**

(51) Int Cl.:
***A01N 43/38*** (2006.01) ***A01N 43/36*** (2006.01)
*A01N 43/38* (2006.01) *A01N 55/00* (2006.01)
*A01N 53/00* (2006.01) *A01N 37/44* (2006.01)
*A01N 37/38* (2006.01) *A01N 31/14* (2006.01)
*A01N 43/36* (2006.01) *A01N 53/00* (2006.01)
*A01N 37/44* (2006.01) *A01N 37/38* (2006.01)
*A01N 31/14* (2006.01)

(86) Internationale Anmeldenummer:
**PCT/EP2001/006841**

(87) Internationale Veröffentlichungsnummer:
**WO 2002/000025 (03.01.2002 Gazette 2002/01)**

(54) **WIRKSTOFFKOMBINATIONEN MIT INSEKTIZIDEN UND AKARIZIDEN EIGENSCHAFTEN**

COMBINATIONS OF ACTIVE INGREDIENTS, WHICH EXHIBIT INSECTICIDAL AND ACARICIDAL PROPERTIES

COMBINAISONS DE PRINCIPES ACTIFS PRESENTANT DES PROPRIETES INSECTICIDES ET ACARACIDES

(84) Benannte Vertragsstaaten:
**AT BE CH CY DE DK ES FI FR GB GR IE IT LI LU MC NL PT SE TR**

(30) Priorität: **29.06.2000 DE 10031765**
**28.07.2000 DE 10036896**

(43) Veröffentlichungstag der Anmeldung:
**09.04.2003 Patentblatt 2003/15**

(73) Patentinhaber: **Bayer CropScience AG**
**40789 Monheim (DE)**

(72) Erfinder:
- **FISCHER, Reiner**
**40789 Monheim (DE)**
- **ERDELEN, Christoph**
**42799 Leichlingen (DE)**

(56) Entgegenhaltungen:
**WO-A-97/36868**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Wirkstoffkombinationen, die aus bekannten cyclischen Ketoenole einerseits und weiteren bekannten insektiziden Wirkstoffen andererseits bestehen und sehr gut zur Bekämpfung von tierischen Schädlingen wie Insekten und unerwünschten Akariden geeignet sind.

Es ist bereits bekannt, dass bestimmte cyclische Ketoenole herbizide, insektizide und akarizide Eigenschaften besitzen. Die Wirksamkeit dieser Stoffe ist gut, lässt aber bei niedrigen Aufwandmengen in manchen Fällen zu wünschen übrig.

Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/ 43 275, WO 98/05 638, WO 98/06 721, WO 98/25 928, WO 99/16 748, WO 99/24 437, WO 99/43 649, WO 99/48 869 und WO 99/55 673).

Weiterhin ist schon bekannt, dass zahlreiche Heterocyclen, Organozinn-Verbindungen, Benzoylharnstoffe und Pyrethroide insektizide und akarizide Eigenschaften besitzen (vgl. WO 93-22 297, WO 93-10 083, DE-A 2 641 343, EP-A-347 488, EP-A-210 487, US-A 3 264 177 und EP-A-234 045). Allerdings ist die Wirkung dieser Stoffe nicht immer befriedigend.

Weiterhin sind Wirkstoffkombinationen mit synergistischen insektiziden und akariziden Eigenschaften bekannt, die ein Dihydrofuranon-Derivat und ein Pyrethroid enthalten (vgl. WO 01/33966 und DE-A-19 939 395).

Es wurde nun gefunden, dass Verbindungen der Formel (I)

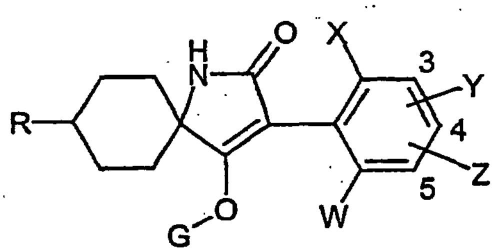

(I)

| Verbindungs Nr. | W | X | Y | Z | R | G | Fp.°C |
|---|---|---|---|---|---|---|---|
| I-1 | H | Br | 5-$CH_3$ | H | $OCH_3$ | CO-i-$C_3H_7$ | 122 |
| I-2 | H | Br | 5-$CH_3$ | H | $OCH_3$ | $CO_2$-$C_2H_5$ | 140 - 142 |
| I-3 | H | $CH_3$ | 5-$CH_3$ | H | $OCH_3$ | H | > 220 |
| I-4 | H | $CH_3$ | 5-$CH_3$ | H | $OCH_3$ | $CO_2$-$C_2H_5$ | 128 |
| I-5 | $CH_3$ | $CH_3$ | 3-Br | H | $OCH_3$ | H | > 220 |
| I-6 | $CH_3$ | $CH_3$ | 3-Cl | H | $OCH_3$ | H | 219 |
| I-7 | H | Br | 4-$CH_3$ | 5-$CH_3$ | $OCH_3$ | CO-i-$C_3H_7$ | 217 |
| I-8 | H | $CH_3$ | 4-Cl | 5-$CH_3$ | $OCH_3$ | $CO_2C_2H_5$ | 162 |
| I-9 | H | $CH_3$ | 4-$CH_3$ | 5-$CH_3$ | $OCH_3$ | 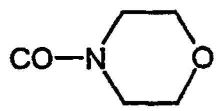 | Öl |
| I-10 | $CH_3$ | $CH_3$ | 3-$CH_3$ | 4-$CH_3$ | $OCH_3$ | H | >220 |
| I-11 | H | $CH_3$ | 5-$CH_3$ | H | $OC_2H_5$ | 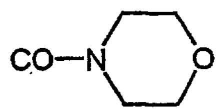 | Öl |
| I-12 | $CH_3$ | $CH_3$ | 3-Br | H | $OC_2H_5$ | CO-i-$C_3H_7$ | 212 - 214 |
| I-13 | H | $CH_3$ | 4-$CH_3$ | 5-$CH_3$ | $OC_2H_5$ | CO-n-Pr | 134 |
| I-14 | H | $CH_3$ | 4-$CH_3$ | 5-$CH_3$ | $OC_2H_5$ | CO-i-Pr | 108 |
| I-15 | H | $CH_3$ | 4-$CH_3$ | 5-$CH_3$ | $OC_2H_5$ | CO-c-Pr | 163 |

und mindestens eine der nach folgenden Pyrethroide

1. Acrinathrin

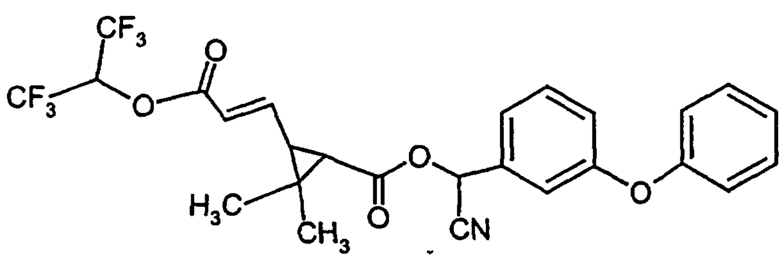

bekannt aus EP-A-048 186
und/oder
2. Alpha-cypermethrin

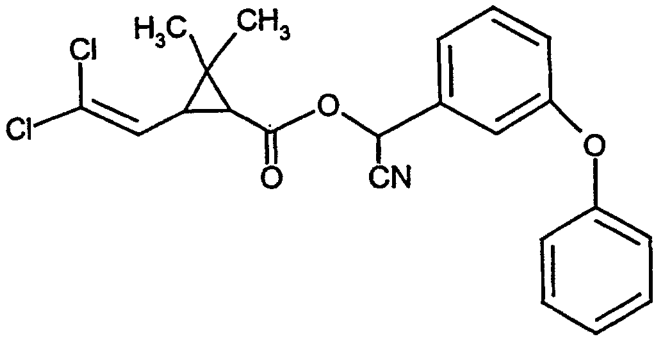

bekannt aus EP-A-067 461
und/oder
3. Betacyfluthrin

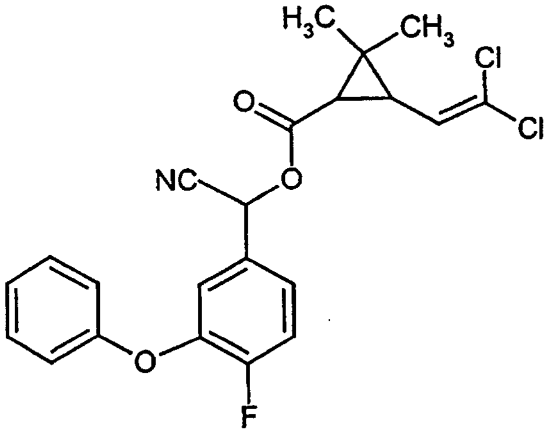

bekannt aus EP-A-206 149
und/oder
4. Cyhalothrin

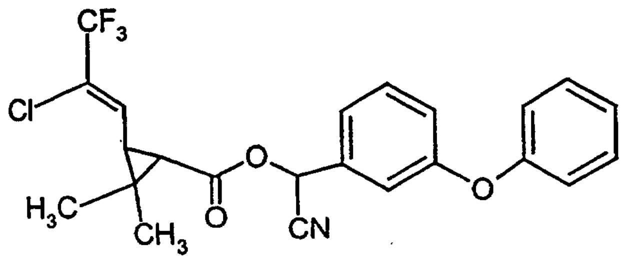

bekannt DE-A-2 802 962
und/oder
5. Cypermethrin

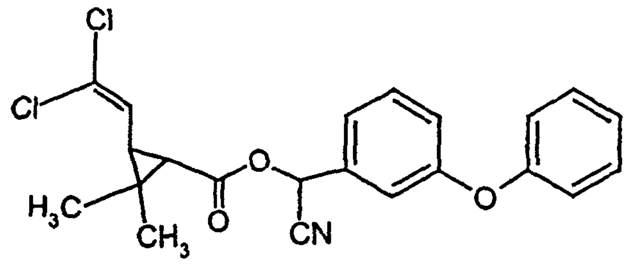

bekannt DE-A-2 326 077
und/oder
6. Deltamethrin

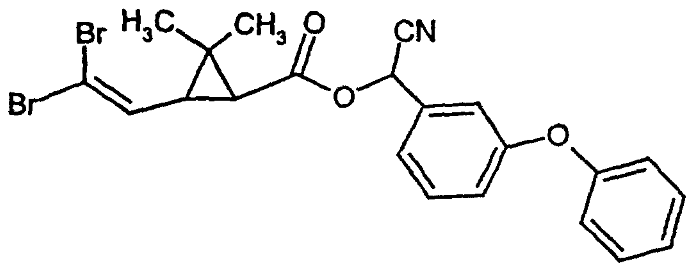

bekannt DE-A-2 326 077
und/oder
7. Esfenvalerat

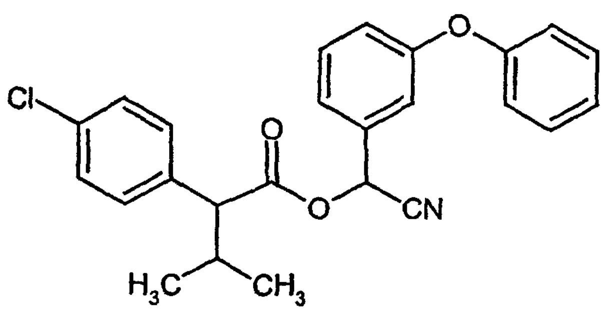

bekannt aus DE-A-2 737 297
und/oder
8. Ethofenprox

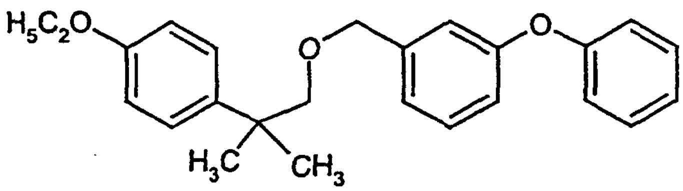

bekannt DE-A-3 117 510

und/oder
9. Fenpropathrin

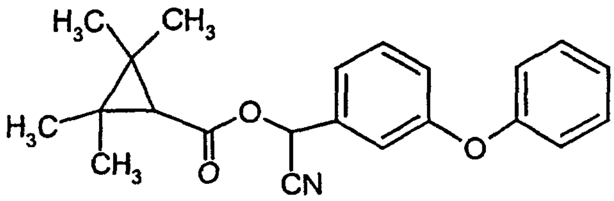

bekannt DE-A-2 231 312
und/oder
10. Fenvalerat

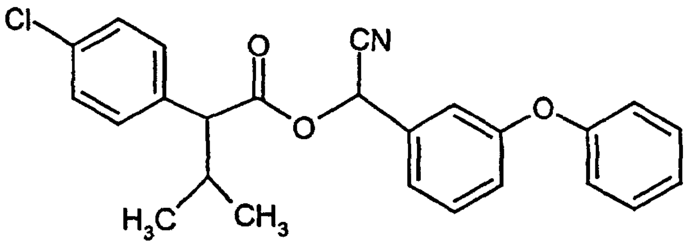

bekannt DE-A-2 335 347
und/oder
11. Flucythrinat

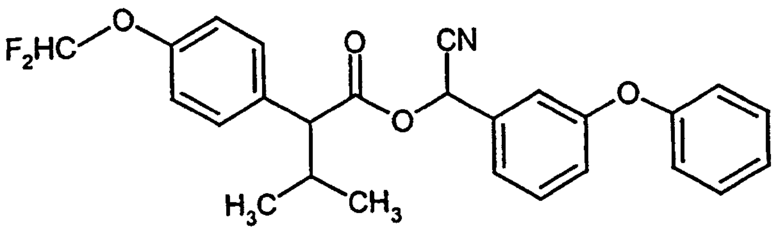

bekannt DE-A-2 757 066
und/oder
12. Lambda-cyhalothrin

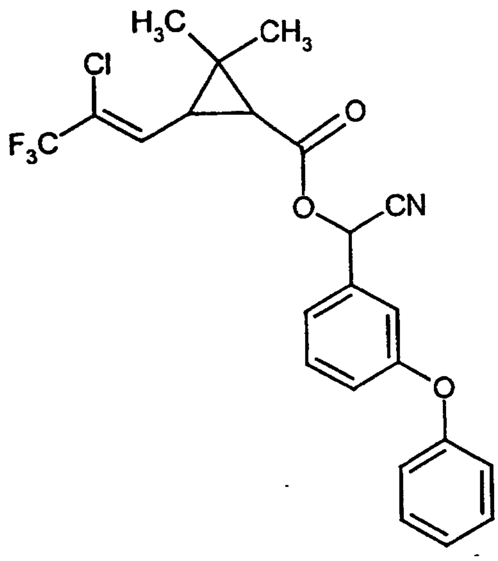

bekannt EP-A-106 469
und/oder
13. Permethrin

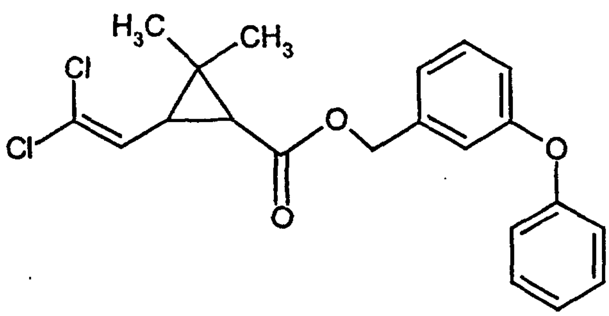

bekannt DE-A-2 326 077
und/oder
14. Taufluvalinat

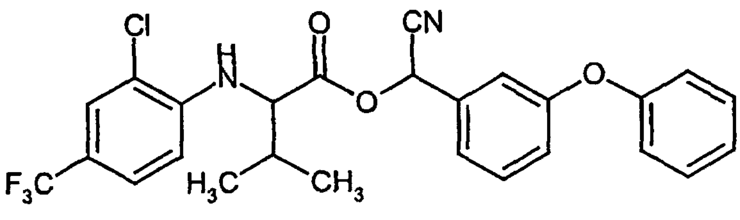

bekannt EP-A-038 617
und/oder
15. Tralomethrin

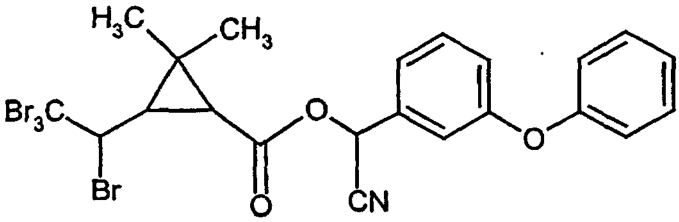

bekannt DE-A-2 742 546
und/oder
16. Zeta-cypermethrin

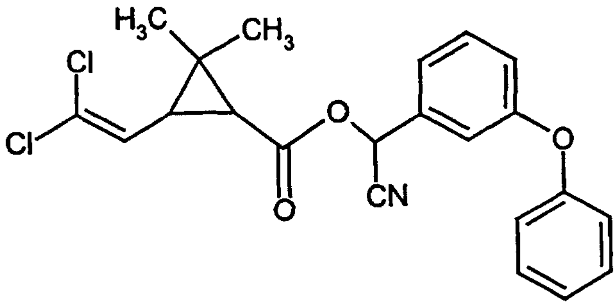

bekannt EP-A-026 542
und/oder
17. Gyfluthrin

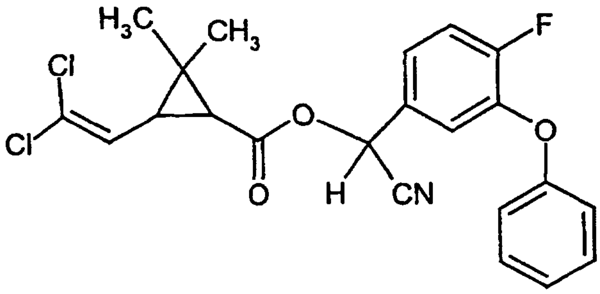

bekannt aus DE-A-27 09 264
und/oder
18. Bifenthrin

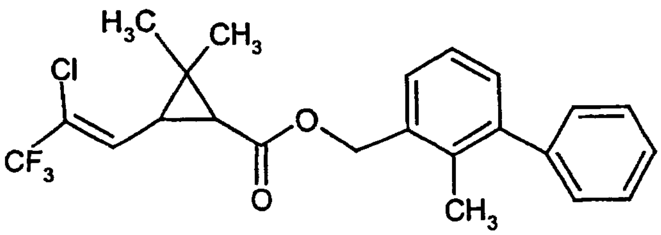

bekannt aus EP-A-049 977
und/oder
19. Cycloprothrin

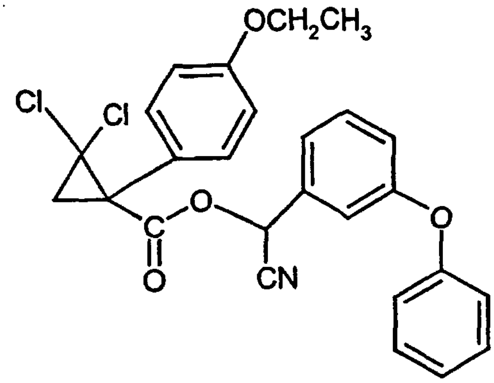

bekannt aus DE-A-2653189
und/oder
20. Eflusilanat

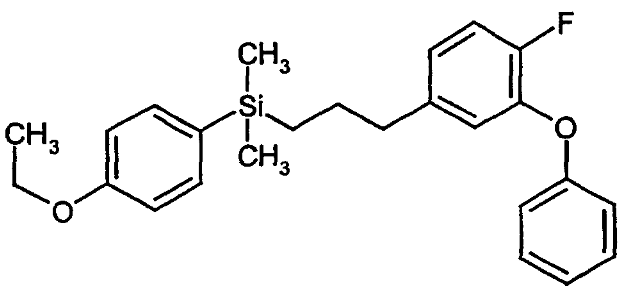

bekannt aus DE-A-36 04 781
und/oder
21. Fubfenprox

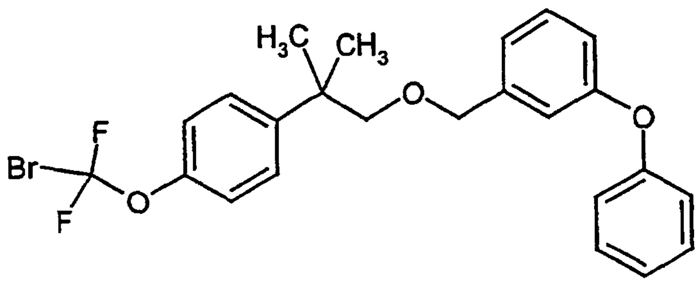

bekannt aus DE-A-37 08 231
und/oder
22. Pyrethrin

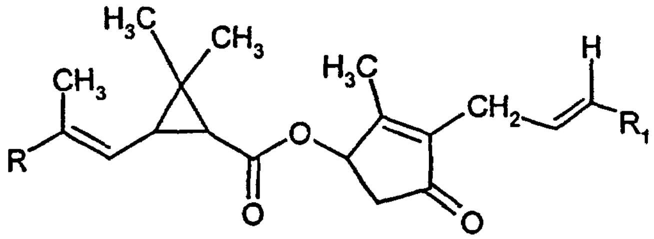

R = $-CH_3$ oder $-CO_2CH_3$
$R_1$ = $-CH=CH_2$ oder $-CH_3$ oder $-CH_2CH_3$
bekannt aus The Pesticide manual, 1997, 11.Ausgabe, S.1056
und/oder
23. Resmethrin

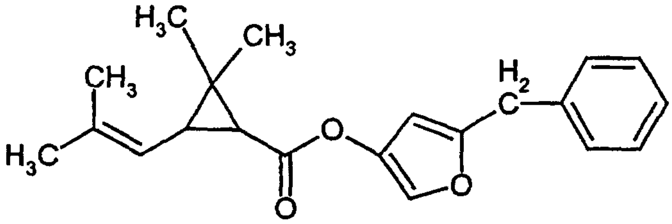

bekant aus GB-A-1 168 797
sehr gute insektizide und akarizide Eigenschaften besitzen.

Überraschenderweise ist die insektizide und akarizide Wirkung der erfindungsgemäßen Wirkstoffkombination wesentlich höher als die Summe der Wirkungen der einzelnen Wirkstoffe. Es liegt ein nicht vorhersehbarer echter synergistischer Effekt vor und nicht nur eine Wirkungsergänzung.

Die erfindungsgemäßen Wirkstoffkombinationen enthalten neben mindestens einem Wirkstoff der Formel (I) mindestens einen Wirkstoff der Verbindungen 1 bis 23.

Die Wirkstoffkombinationen können darüber hinaus auch weitere fungizid, akarizid oder insektizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im allgemeinen enthalten die erfindungsgemäßen Kombinationen Wirkstoffe der Formel (I) und den Mischpartner in den in der nachfolgenden Tabelle angegeben bevorzugten und besonders bevorzugten Mischungsverhältnissen:
* die Mischungsverhältnisse basieren auf Gewichtsverhältnissen. Das Verhältnis ist zu verstehen als Wirkstoff der Formel (I):Mischpartner

| Mischpartner | bevorzugtes Mischungsverhältnis | besonders bevorzugtes Mischungsverhältnis |
|---|---|---|
| Acrinathrin | 20:1 bis 1:50 | 10:1 bis 1:1 |
| Alpha-Cypermethrin | 50:1 bis 1:5 | 10:1 bis 1:1 |
| Betacyfluthrin | 50:1 bis 1:5 | 10:1 bis 1:1 |
| Cyhalothrin | 50:1 bis 1:5 | 10:1 bis 1:1 |
| Cypermethrin | 50:1 bis 1:5 | 10:1 bis 1:1 |
| Deltamethrin | 50:1 bis 1:5 | 10:1 bis 1:1 |
| Esfenvalerat | 50:1 bis 1:5 | 10:1 bis 1:1 |

Tabelle fortgesetzt

| Mischpartner | bevorzugtes Mischungsverhältnis | besonders bevorzugtes Mischungsverhältnis |
|---|---|---|
| Etofenprox | 10:1 bis 1:10 | 5:1 bis 1:5 |
| Fenpropathrin | 10:1 bis 1:10 | 5:1 bis 1:5 |
| Fenvalerat | 20:1 bis 1:5 | 10:1 bis 1:1 |
| Flucythrinat | 50:1 bis 1:5 | 10:1 bis 1:1 |
| Lambda-Cyhalothrin | 50:1 bis 1:5 | 10:1 bis 1:1 |
| Permethrin | 10:1 bis 1:10 | 5:1 bis 1:5 |
| Tau-fluvalinat | 20:1 bis 1:5 | 10:1 bis 1:2 |
| Tralomethrin | 50:1 bis 1:5 | 10:1 bis 1:1 |
| Zeta-Cypermethrin | 50:1 bis 1:5 | 10:1 bis 1:2 |
| Cyfluthrin | 50:1 bis 1:5 | 10:1 bis 1:1 |
| Bifenthrin | 10:1 bis 1:10 | 10:1 bis 1:1 |
| Cycloprothrin | 10:1 bis 1:10 | 5:1 bis 1:5 |
| Eflusilanat | 10:1 bis 1:10 | 5:1 bis 1:5 |
| Fubfenprox | 10:1 bis 1:10 | 5:1 bis 1:5 |
| Pyrethrin | 50:1 bis 1:10 | 5:1 bis 1:1 |
| Resmethrin | 50:1 bis 1:10 | 5:1 bis 1:1 |

Die erfindungsgemäßen Wirkstoffkombinationen eignen sich zur Bekämpfung von tierischen Schädlingen, vorzugsweise Arthropoden und Nematoden, insbesondere Insekten und Spinnentieren, die in der Landwirtschaft, der Tiergesundheit, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp.,

Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyanin farbstoffe und Spurennährstoffe wie Salze von Eisen, Mangan, Bor, Kupfer, Kobalt, Molybdän und Zink verwendet weiden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Auch eine Mischung mit anderen bekannten Wirkstoffen, wie Herbiziden oder mit Düngemitteln und Wachstumsregulatoren ist möglich.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die erfindungsgemäßen Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophoreis spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffkombinationen eine wirtschaftlichere und einfachere Tierhaltung möglich ist.

Die Anwendung der erfindungsgemäßen Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Fornikörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
Sirex juvencus, Urocerus gigas, Urocerus gigas taignus, Urocerus augur.
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit

einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Gemisch in diesem Lösungsmittelgemisch löslich oder emulgierbar ist

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Zugleich können die erfindungsgemäßen Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Wirkstoffkombinationen eine hervorragende Antifouling (Antibewuchs)-Wirkung aufweisen.

Durch Einsatz der erfindungsgemäßen Wirkstoffkombinationen kann auf den Einsatz von Schwermetallen wie z.B. in Bis(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie
2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoffkombinationen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind*. **1985,** *37,* 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharae in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae,

Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Mernbranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Erfindungsgemäß können alle Planzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft, Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhiozome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise emiedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Emteprodukte möglich, die über die eigentlich zu erwartenden Effekte hinausgehen.

Zu den bevorzugten erfindungsgemäß zu behandelnden transgenen (gentechnologisch erhaltenen) Pflanzen

bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den erfindungsgemäßen Wirkstoffmischungen behandelt werden. Die bei den Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die gute insektizide und akarizide Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt bei Insektiziden und Akariziden immer dann vor, wenn die Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann nach S.R Colby, Weeds 15 (1967), 20-22) wie folgt berechnet werden:

Wenn

- X den Abtötungsgrad, ausgedrückt in % der unbehandelten Kontrolle, beim Einsatz des Wirkstoffes A in einer Aufwandmenge von m g/ha oder in einer Konzentration von m ppm bedeutet,
- Y den Abtötungsgrad, ausgedrückt in % der unbehandelten Kontrolle, beim Einsatz des Wirkstoffes B in einer Aufwandmenge von n g/ha oder in einer Konzentration von n ppm bedeutet und
- E den Abtötungsgrad, ausgedrückt in % der unbehandelten Kontrolle, beim Einsatz der Wirkstoffe A und B in Aufwandmengen von m und n g/ha oder in einer Konzentration von m und n ppm bedeutet,

dann ist

$$E = X + Y - \frac{X \cdot Y}{100}$$

Ist der tatsächliche insektizide Abtötungsgrad größer als berechnet, so ist die Kombination in ihrer Abtötung überadditiv, d.h. es liegt ein synergistischer Effekt vor. In diesem Fall muß der tatsächlich beobachtete Abtötungsgrad größer sein als der aus der oben angeführten Formel errechnete Wert für den erwarteten Abtötungsgrad (E).

Nach der gewünschten Zeit wird die Abtötung in % bestimmt. Dabei bedeutet 100 %, dass alle Tiere abgetötet wurden; 0 % bedeutet, dass keine Tiere abgetötet wurden.

## Anwendungsbeispiele

### Beispiel A

**Aphis gossypii-Test**

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.
Baumwollblätter (Gossypium hirsutum), die stark von der Baumwollblattlaus (Aphis Gossypii) befallen sind, werden durch Tauchen in die Wirkstoffzubereitung der gewünschten Konzentration behandelt.
Nach der gewünschten Zeit wird die Abtötung in % bestimmt. Dabei bedeutet 100 %, dass alle Tiere abgetötet wurden; 0 % bedeutet, dass keine Blattläuse abgetötet wurden. Die ermittelten Abtötungswerte verrechnet man nach der Colby-Formel.
Bei diesem Test zeit z.B. die folgende Wirkstoffkombination gemäß vorliegender Anmeldung eine synergistisch verstärkte Wirksamkeit im Vergleich zu den einzeln angewendeten Wirkstoffen:

### Tabelle A

pflanzenschädigende Insekten

**Aphis gossypii-Test**

| Wirkstoffe | Wirkstoffkonzentration in ppm | Abtötungsgrad in % nach $6^d$ |
|---|---|---|
| Bsp. I-10 bekannt aus WO 97/36868 | 1,6 | 0 |
| Cyfluthrin bekannt | 1,6 | 75 |
| Bsp. I-10+Cyfluthrin (1:1) erfindungsgemäß | 1,6 + 1,6 | gef.* ber.** 95 75 |
| * gef. = gefundene Wirkung<br>** ber. = nach der Colby-Formel berechnete Wirkung | | |

### Beispiel B

**Myzus-Test**

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.
Kohlblätter (Brassica oleracea), die stark von der Pfirsichblattlaus (Myzus persicae) befallen sind, werden durch Tauchen in die Wirkstoffzubereitung der gewünschten Konzentration behandelt.
Nach der gewünschten Zeit wird die Abtötung in % bestimmt. Dabei bedeutet 100 %, dass alle Tiere abgetötet

wurden; 0 % bedeutet, dass keine Tiere abgetötet wurden. Die ermittelten Abtötungswerte verrechnet man nach der Colby-Formel.

Bei diesem Test zeit z.B. die folgende Wirkstoffkombination gemäß vorliegender Anmeldung eine synergistisch verstärkte Wirksamkeit im Vergleich zu den einzeln angewendeten Wirkstoffen:

**Tabelle B**

pflanzenschädigende Insekten

**Myzus-Test**

| Wirkstoffe | Wirkstoffkonzentration in ppm | Abtötungsgrad in % nach 1$^{d}$ |
|---|---|---|
| Bsp. I-10 bekannt aus WO 97/36868 | 1,6 | 0 |
| Cyfluthrin bekannt | 1,6 | 25 |
| Bsp. I-10 + Cyfluthrin (1:1) erfindungsgemäß | 1,6 + 1,6 | gef.* 65 ber.** 25 |
| * gef. = gefundene Wirkung<br>** ber. = nach der Colby-Formel berechnete Wirkung | | |

**Beispiel C**

**Grenzkonzentrations-Test / Bodeninsekten - Behandlung transgener Pflanzen**

Testinsekt: **Diabrotica balteata - Larven im Boden**
Lösungsmittel: 7 Gewichtsteile Aceton
Emulgator. 1 Gewichtsteil Alkylarylpolyglykolether

Zur Herstellung einer zweclanäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

**Beispiel D**

**Heliothis virescens - Test - Behandlung transgener Pflanzen**

Lösungsmittel: 7 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch

Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Mittel entaltend Mischüngen aus Verbindungen der Formel (I)

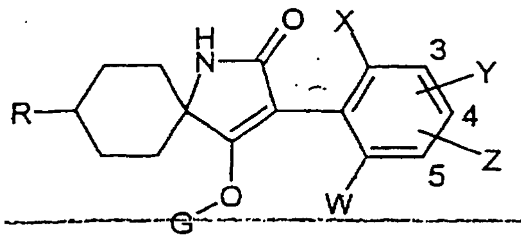

(I)

in welcher
W, X, Y, Z, R und G die in der Tabelle angegebenen Bedeutungen haben

| W | X | Y | Z | R | G |
|---|---|---|---|---|---|
| H | Br | 5-$CH_3$ | H | $OCH_3$ | CO-i-$C_3H_7$ |
| H | Br | 5-$CH_3$ | H | $OCH_3$ | $CO_2$-$C_2H_5$ |
| H | $CH_3$ | 5-$CH_3$ | H | $OCH_3$ | H |
| H | $CH_3$ | 5-$CH_3$ | H | $OCH_3$ | $CO_2$-$C_2H_5$ |
| $CH_3$ | $CH_3$ | 3-Br | H | $OCH_3$ | H |
| $CH_3$ | $CH_3$ | 3-Cl | H | $OCH_3$ | H |
| H | Br | 4-$CH_3$ | 5-$CH_3$ | $OCH_3$ | CO-i-$C_3H_7$ |
| H | $CH_3$ | 4-Cl | 5-$CH_3$ | $OCH_3$ | $CO_2C_2H_5$ |
| H | $CH_3$ | 4-$CH_3$ | 5-$CH_3$ | $OCH_3$ | 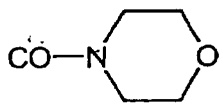 |
| $CH_3$ | $CH_3$ | 3-$CH_3$ | 4-$CH_3$ | $OCH_3$ | H |
| H | $CH_3$ | 5-$CH_3$ | H | $OC_2H_5$ | 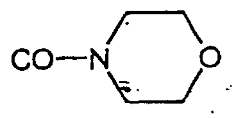 |
| $CH_3$ | $CH_3$ | 3-Br | H | $OC_2H_5$ | CO-i-$C_3H_7$ |
| H | $CH_3$ | 4-$CH_3$ | 5-$CH_3$ | $OC_2H_5$ | CO-n-Pr |
| H | $CH_3$ | 4-$CH_3$ | 5-$CH_3$ | $OC_2H_5$ | CO-i-Pr |
| H | $CH_3$ | 4-$CH_3$ | 5-$CH_3$ | $OC_2H_5$ | CO-c-Pr |

und mindestens eine der nachfolgenden Verbindungen
Acrinathrin
Alpha-Cypermethrin

Betacyfluthrin
Cyhalothrin
Cypermethrin
Deltamethrin
Esfenvalerat
Etofenprox
Fenpropathrin
Fenvalerat
Flucythrinat
Lambda-Cyhalothrin
Permethrin
Tau-fluvalinat
Tralomethrin
Zeta-Cypermethrin
Cyfluthrin
Bifenthrin
Cycloprothrin
Eflusilanat
Fubfenprox
Pyrethrin
Resmethrin.

**2.** Verwendung von Mischungen, wie in Anspruch 1, definiert, zur Bekämpfung tierischer Schädlinge.

**3.** Verfahren zur Bekämpfung tierischer Schädlinge, **dadurch gekennzeichnet, dass** man Mischungen, wie in Anspruch 1, definiert, auf tierische Schädlinge und/oder deren Lebensraum einwirken lässt.

**4.** Verfahren zur Herstellung insektizider und akarizider Mittel, **dadurch gekennzeichnet, dass** man Mischungen, wie in Anspruch 1, definiert, mit Streckmitteln und/oder oberflächenaktiven Stoffen vermischt.

**Claims**

**1.** Composition comprising mixtures of compounds of the formula (I)

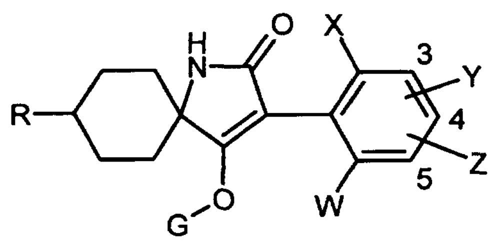

(I)

in which
W, X, Y, Z, R and G are each as defined in the table

| W | X | Y | Z | R | G |
|---|---|---|---|---|---|
| H | Br | $5-CH_3$ | H | $OCH_3$ | $CO-i-C_3H_7$ |
| H | Br | $5-CH_3$ | H | $OCH_3$ | $CO_2-C_2H_5$ |
| H | $CH_3$ | $5-CH_3$ | H | $OCH_3$ | H |
| H | $CH_3$ | $5-CH_3$ | H | $OCH_3$ | $CO_2-C_2H_5$ |
| $CH_3$ | $CH_3$ | 3-Br | H | $OCH_3$ | H |
| $CH_3$ | $CH_3$ | 3-Cl | H | $OCH_3$ | H |
| H | Br | $4-CH_3$ | $5-CH_3$ | $OCH_3$ | $CO-i-C_3H_7$ |
| H | $CH_3$ | 4-Cl | $5-CH_3$ | $OCH_3$ | $CO_2C_2H_5$ |
| H | $CH_3$ | $4-CH_3$ | $5-CH_3$ | $OCH_3$ | 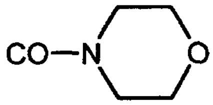 |
| $CH_3$ | $CH_3$ | $3-CH_3$ | $4-CH_3$ | $OCH_3$ | H |

| W | X | Y | Z | R | G |
|---|---|---|---|---|---|
| H | $CH_3$ | $5-CH_3$ | H | $OC_2H_5$ | 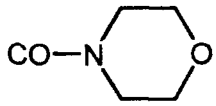 |
| $CH_3$ | $CH_3$ | 3-Br | H | $OC_2H_5$ | $CO-i-C_3H_7$ |
| H | $CH_3$ | $4-CH_3$ | $5-CH_3$ | $OC_2H_5$ | CO-n-Pr |
| H | $CH_3$ | $4-CH_3$ | $5-CH_3$ | $OC_2H_5$ | CO-i-Pr |
| H | $CH_3$ | $4-CH_3$ | $5-CH_3$ | $OC_2H_5$ | CO-c-Pr |

and at least one of the compounds below
acrinathrin
alpha-cypermethrin
betacyfluthrin
cyhalothrin
cypermethrin
deltamethrin
esfenvalerate
etofenprox
fenpropathrin
fenvalerate
flucythrinate
lambda-cyhalothrin
permethrin
tau-fluvalinate
tralomethrin
zeta-cypermethrin
cyfluthrin
bifenthrin
cycloprothrin

eflusilanate
fubfenprox
pyrethrin
resmethrin.

**2.** Use of mixtures as defined in Claim 1 for controlling animal pests.

**3.** Method for controlling animal pests, **characterized in that** mixtures as defined in Claim 1 are allowed to act on animal pests and/or their habitat.

**4.** Process for preparing insecticidal and acaricidal compositions, **characterized in that** mixtures as defined in Claim 1 are mixed with extenders and/or surfactants.

## Revendications

**1.** Compositions contenant des mélanges de composés de formule (I)

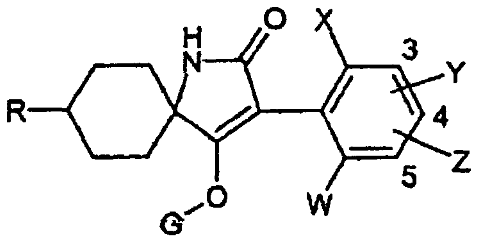

(I)

dans laquelle
W, X, Y, Z, R et G ont les définitions indiquées dans le tableau

| W | X | Y | Z | R | G |
|---|---|---|---|---|---|
| H | Br | 5-$CH_3$ | H | $OCH_3$ | CO-i-$C_3H_7$ |
| H | Br | 5-$CH_3$ | H | $OCH_3$ | $CO_2$-$C_2H_5$ |
| H | $CH_3$ | 5-$CH_3$ | H | $OCH_3$ | H |
| H | $CH_3$ | 5-$CH_3$ | H | $OCH_3$ | $CO_2$-$C_2H_5$ |
| $CH_3$ | $CH_3$ | 3-Br | H | $OCH_3$ | H |
| $CH_3$ | $CH_3$ | 3-Cl | H | $OCH_3$ | H |
| H | Br | 4-$CH_3$ | 5-$CH_3$ | $OCH_3$ | CO-i-$C_3H_7$ |
| H | $CH_3$ | 4-Cl | 5-$CH_3$ | $OCH_3$ | $CO_2C_2H_5$ |
| H | $CH_3$ | 4-$CH_3$ | 5-$CH_3$ | $OCH_3$ | 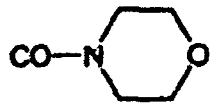 |
| $CH_3$ | $CH_3$ | 3-$CH_3$ | 4-$CH_3$ | $OCH_3$ | H |
| H | $CH_3$ | 5-$CH_3$ | H | $OC_2H_5$ | 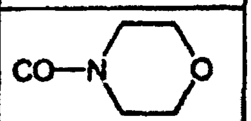 |
| $CH_3$ | $CH_3$ | 3-Br | H | $OC_2H_5$ | CO-i-$C_3H_7$ |
| H | $CH_3$ | 4-$CH_3$ | 5-$CH_3$ | $OC_2H_5$ | CO-n-Pr |
| H | $CH_3$ | 4-$CH_3$ | 5-$CH_3$ | $OC_2H_5$ | CO-i-Pr |
| H | $CH_3$ | 4-$CH_3$ | 5-$CH_3$ | $OC_2H_5$ | CO-c-Pr |

et au moins l'un des composés suivants :

acrinathrine,
alpha-cyperméthrine,
bétacyfluthrine,
cyhalothrine,
cyperméthrine,
deltaméthrine,
esfenvalérate,
étofenprox,
fenpropathrine,
fenvalérate,
flucythrinate,
lambda-cyhalothrine,
perméthrine,
tau-fluvalinate,
tralométhrine,
zéta-cyperméthrine,
cyfluthrine,
bifenthrine,
cycloprothrine,
éflusilanate,
fubfenprox,

pyréthrine,
restméthrine.

2. Utilisation de mélanges tels que définis dans la revendication 1, pour combattre des parasites animaux.

3. Procédé pour combattre des parasites animaux, **caractérisé en ce qu'**on fait agir des mélanges tels que définis dans la revendication 1 sur des parasites animaux et/ou sur leur milieu.

4. Procédé de préparation de compositions insecticides et acaricides, **caractérisé en ce qu'**on mélange avec des diluants et/ou des agents tensioactifs des mélanges tels que définis dans la revendication 1.